Europäisches Patentamt

European Patent Office

Office européen des brevets

(11) Veröffentlichungsnummer: **0 258 542**
**A1**

## (12) EUROPÄISCHE PATENTANMELDUNG

(21) Anmeldenummer: **87107869.7**

(22) Anmeldetag: **30.05.87**

(51) Int. Cl.⁴: **A61M 5/14**

(30) Priorität: **06.08.86 DE 8621044 U**

(43) Veröffentlichungstag der Anmeldung:
**09.03.88 Patentblatt 88/10**

(84) Benannte Vertragsstaaten:
**AT BE CH DE ES FR GB GR IT LI NL SE**

(71) Anmelder: **B. Braun Melsungen AG**
**Carl-Braun Strasse**
**D-3508 Melsungen(DE)**

(72) Erfinder: **Schmidt, Klaus-Joachim**
**Brandenburgerstrasse 16**
**D-3501 Ahnatal(DE)**

(74) Vertreter: **Selting, Günther, Dipl.-Ing. et al**
**Deichmannhaus am Hauptbahnhof**
**D-5000 Köln 1(DE)**

(54) **Punktionsbesteck.**

㉗ Das Punktionsbesteck (10) besteht aus einem Kunststoffkapillar (11), das an seinem patientenfernen Ende einen hohlen Ansatzstutzen (12) aufweist, aus einer in das Kunststoffkapillar (11) einschiebbaren Stahlkanüle (16) mit einer angeschliffenen Spitze (17) und einem Kanülenansatz (15), der mit dem hohlen Ansatzstutzen (12) lösbar zusammensteckbar ist und aus einer hülsenartigen Schutzkappe (22) für die langgestreckten Teile. Die Schutzkappe (22) weist einen geschlossenen, dicken Boden (23) auf, in den die angeschliffene Spitze (17) der Stahlkanüle (16) klemmend einstechbar ist, wodurch eine feste Verbindung zwischen diesen beiden Teilen entsteht. Die Stahlkanüle (16) kann in die üblichen Entsorgungsbehältnisse abgelegt werden, ohne daß die Schutzkappe (22) abfällt.

EP 0 258 542 A1

FIG.1

FIG.2

## Punktionsbesteck

Die Erfindung bezieht sich auf ein Punktionsbesteck, bestehend aus einem Kunststoffkapillar, das an seinem patientenfernen Ende einen hohlen Ansatzstutzen aufweist, aus einer in das Kunststoffkapillar einschiebbaren Stahlkanüle mit einer angeschliffenen Spitze und einem Kanülenansatz, der mit dem hohlen Ansatzstutzen lösbar zusammensteckbar ist und aus einer hülsenartigen Schutzkappe für die langgestreckten Teile.

Bei einem solchen Punktionsbesteck bewirkt die über das stumpfe Ende der Kunststoffkapillare vorstehende angeschliffene Spitze der Stahlkanüle die Punktion eines Blutgefäßes und das Kunststoffkapillar dient nach Herausziehen der Stahlkanüle als Verweilkatheter, der in dem Blutgefäß verbleibt und an dessen hohlen Ansatzstutzen ein Überleitungsgerät zur Infusion oder Transfusion von Flüssigkeiten angeschlossen wird. Die freie Stahlkanüle wird weggeworfen. Damit das Behandlungs und Entsorgungspersonal sich nicht durch Stichverletzungen z.B. mit Hepatitis oder AIDS, infizieren kann, ist es erwünscht, daß die angeschliffene Spitze der relativ großkalibrigen Stahlkanüle durch Aufstecken der Schutzkappe beim Wegwerfen geschützt wird. Voraussetzung für einen solchen Schutz ist allerdings eine gesicherte Festhaltung der Schutzkappe an der Stahlkanülenanordnung. Hierbei ergeben sich gewisse Probleme. Bei dem benutzungsbereiten Punktionsbesteck wird das offene eine Ende der Schutzkappe auf einen angepaßten Fortsatz des hohlen Ansatzstutzens des Kunststoffkapillars klemmend aufgesteckt und das andere Ende, das offen oder durch einen Boden verschlossen sein kann, ragt über die angeschliffene Spitze der Stahlkanüle ein Stück hinaus, so daß sie abgedeckt ist. Bei Punktionsbestecken mit kurzem hohlen Ansatzstutzen des Kunststoffkapillars paßt die Schutzkappe ohne nennenswerte Verlängerung auch auf die herausgezogene Stahlkanüle, wenn ihr offenes Ende auf den Fortsatz des Kanülenansatzes klemmend aufgesteckt ist. Allerdings ist die Haltbarkeit dieser Verbindung von der Einhaltung sehr enger Toleranzen bei der Herstellung der Teile abhängig und es wird selbst die an sich anspruchslos ausgebildete Schutzkappe zu einem verteuerten Spezialteil.Bei Punktionsbestecken, deren hohler Ansatzstutzen des Kunststoffkapillars Fixierplatten zur Befestigung auf der Haut eines Patienten aufweist, besteht der Nachteil, daß die Schutzkappe beträchtlich kürzer als die Stahlkanüle ist, so daß sie nach Herausziehen der Stahlkanüle aus dem Kunststoffkapillar zur Verbindung nicht mehr mit dem Fortsatz des Kanülenansatzes zusammensteckbar ist. Um die Schutzkappe nach der Punktion zum Verwerfen der Stahlkanüle auf diese aufstecken zu können, muß sie eine zusätzliche Länge in der Größenordnung der Fixierplatten-Längserstreckung aufweisen. Dies bedeutet jedoch eine Erhöhung des Verpackungs- und Transportvolumens solcher Punktionsbestecke von ca. 20% und damit eine unangemessene Verteuerung.

Der Erfindung liegt die Aufgabe zugrunde, ein Punktionsbesteck der eingangs erwähnten Art so auszubilden, daß die Schutzkappe mit der ineinandersteckenden Einheit von Kunststoffkapillar und Stahlkanüle sich unabhängig von der Länge der herausgezogenen Punktionskanüle mit dieser zur Entsorgung fest verbinden läßt.

Diese Aufgabe wird erfindungsgemäß dadurch gelöst, daß die Schutzkappe einen geschlossenen dicken Boden aufweist, in den die angeschliffene Spitze der Stahlkanüle klemmend einstechbar ist.

Zur Verbindung der aus dem Kunststoffkapillar herausgezogenen Stahlkanüle mit der Schutzkappe wird nun die angeschliffene Spitze der Stahlkanüle ausgenutzt und die Tatsache, daß die der Länge des Kunststoffkapillars angepaßte Länge der Schutzkappe für die herausgezogene Stahlkanüle zu kurz ist, wird für die Verbindung beider Teile bedeutungslos. Das schliffseitige Ende der Schutzkappe ist gemäß der Erfindung so ausgebildet, daß der Schliff der Stahlkanüle nach der Verwendung fest in den dicken Boden eingeschoben werden kann, wodurch eine feste Verbindung zwischen diesen beiden Teilen entsteht. Die Stahlkanüle kann in die üblichen Entsorgungsbehältnisse abgelegt werden, ohne daß die Schutzkappe abfällt. Dem steigenden Sicherheitsbedürfnis zur Vermeidung von Infektionen, wie z.B. AIDS, wird auf diese Weise voll Rechnung getragen. Insbesondere bei Punktionsbestecken mit Fixierplatten wirkt sich die erfindungsgemäße Verbesserung aus, weil das Verpackungs-und Transportvolumen des Punktionsbestecks nicht durch über lange Schutzkappen vergrößert wird und sich keine hierdurch hervorgerufenen Verteuerungen ergeben.

In vorteilhafter Ausgestaltung der Erfindung ist vorgesehen, daß die Tiefe des dicken Bodens mindestens etwa der einfachen und maximal etwa der dreifachen Länge des Schliffes der Spitze entspricht. Die Stahlkanülenschliffe sind sehr scharf und lassen sich leicht in den verwendeten Kunststoff der Schutzkappe eindrücken, wobei durch die angegebene Bemessung der Bodendicke gewährleistet ist, daß die Spitze den Boden nicht durchdringt.

Die Innenfläche des Bodens kann unprofiliert eben sein. Zur Vermeidung des seitlichen Austritts des Stahlkanülenschliffs aus dem dünnen Mantel der Schutzkappe empfiehlt sich jedoch eine Zentrierung der angeschliffenen Spitze beim Einstechen. Dies kann dadurch geschehen, daß die Innenfläche des Bodens kegelförmig spitz vertieft ausgebildet ist. Die auf der Mittelachse der Schutzkappe liegende oder seitlich zu dieser versetzte Spitze der Bodenvertiefung bewirkt zwangsläufig eine etwa zentrale Einführung der Stahlkanülenspitze in den verdickten Schutzkappenboden.

Zweckmäßigerweise ist die spitze Vertiefung auf ihrer Wand mit axialen Klemmrippen versehen, die den lichten Querschnitt der Vertiefung symmetrisch verengen. Die Klemmrippen können gegen die Mittelachse der Schutzkappe gewölbte massive Stege sein, die zwischen sich einen engen Kanal zur klemmenden Aufnahme und Halterung des angeschliffenen Endes der Stahlkanüle bilden. Eine ausreichende Führung und Halterung der Spitze der Stahlkanüle wird durch drei gleichmäßig über den Umfang der Spitzenvertiefung verteilte Klemmrippen erzielt.

In weiterer vorteilhafter Ausgestaltung der Erfindung kann die Innenfläche des Bodens exzentrisch keilförmig vertieft sein, um eine Haltewirkung zwischen Stahlkanüle und Schutzkappe zu erzeugen. In diesem Falle gleitet die Stahlkanüle auf der Innenwand der Schutzkappe entlang in die exzentrische keilförmige Vertiefung hinein und ihre scharfe Spitze dringt exzentrisch in das Fleisch des dicken Bodens der Schutzkappe ein. In allen Fällen wird ein sicherer Halt zwischen Schutzkappe und Punktionskanüle erzielt, ohne daß das offene Ende der Schutzkappe mit einem Fortsatz des Kanülenansatzes zusammengesteckt ist.

Die Schutzkappe ist im Spritzgußverfahren einteilig als rohrförmiger Körper mit äußeren axialen Versteifungsrippen hergestellt. Zwischen den Versteifungsrippen sind in der Wand der Schutzkappe Begasungsöffnungen angeordnet. Diese ermöglichen eine Sterilisation der wegzuwerfenden Stahlkanüle mit Hilfe von Äthylenoxidgas bei aufgesetzter Schutzkappe. Auch hierdurch wird die Sicherheit bei der Entsorgung der Stahlkanülen erhöht.

In der Zeichnung sind Ausführungsbeispiele der Erfindung schematisch dargestellt. Es zeigen

Fig. 1 ein komplettes Punktionsbesteck in Draufsicht,

Fig. 2 eine aus dem Kunststoffkapillar herausgezogene Stahlkanüle mit Schutzkappe in Draufsicht,

Fig. 3, 3a eine weitere Ausführungsform des Schutzkappenbodens im Längsschnitt und im Querschnitt,

Fig. 4, 4a eine dritte Ausführungsform des Schutzkappenbodens im Längsschnitt und im Querschnitt, und

Fig. 5, 5a eine vierte Ausführungsform des Schutzkappenbodens im Längsschnitt und im Querschnitt.

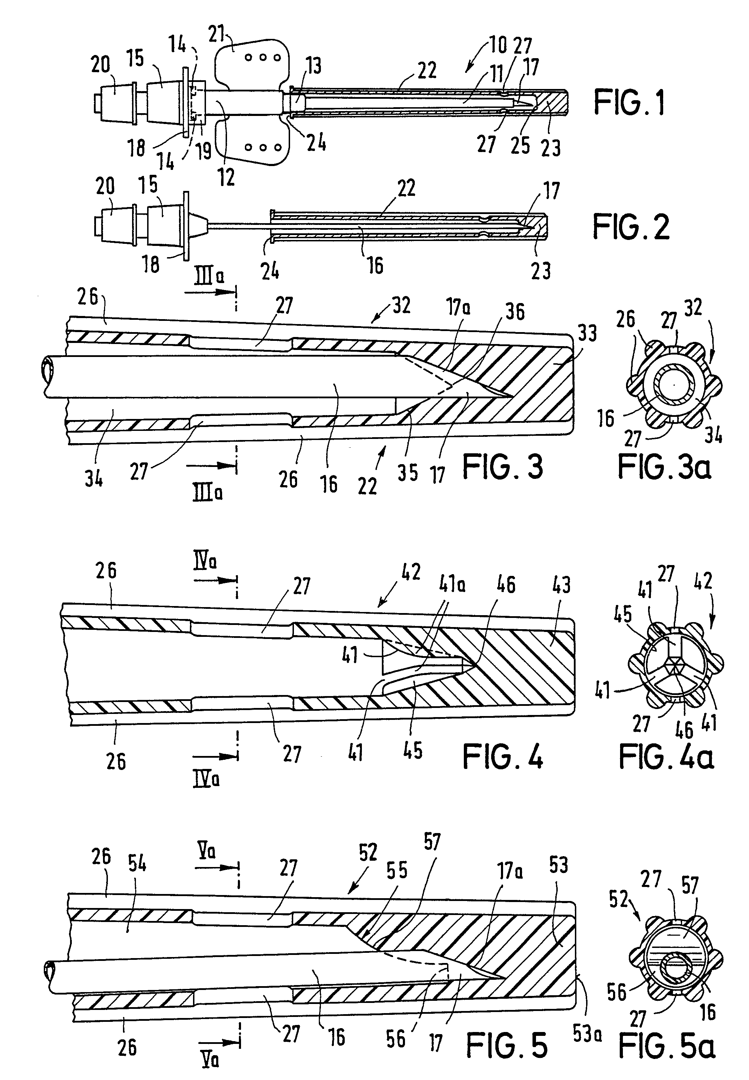

Das Punktionsbesteck 10 besteht im wesentlichen aus einem flexiblen Kunststoffkapillar 11, das an einem Ende an einem hohlen Ansatzstutzen 12 befestigt ist, der an dem kapillarseitigen Ende einen Fortsatz 13 aufweist und an dem Rand des gegenüberliegenden Endes mit zwei radialen Vorsprüngen 14 versehen ist, die in Umfangsrichtung keilförmig verlaufen. Der Hohlraum des Ansatzstutzens 12 ist als Innenkonus zur Aufnahme von Anschlußteilen ausgebildet. Ein solches Anschlußteil befindet sich an einem Kanülenansatz 15, der eine koaxiale Stahlkanüle 16 trägt, deren angeschliffene scharfe Spitze 17 in Fig. 1 über das stumpfe Ende der Kunststoffkapillare 11 vorsteht. Von dem Kanülenansatz 12 steht rechtwinklig eine Griffplatte 18 ab, an die sich auf einer Seite ein Hülsenteil 19 anschließt, der in zusammengestecktem Zustand des Ansatzstutzens 12 und des Kanülenansatzes 15 das Ende des Ansatzstutzens 12 umgibt, wobei die radialen Vorsprünge 14 in entsprechenden Längsaussparungen des Hülsenteils 19 aufgenommen sind. Auf das patientenferne Ende des Kanülenansatzes 15 ist eine Verschlußkappe, insbesondere ein Blutfängerstopfen 20, aufgesteckt. Von dem Ansatzstutzen 12 gehen zwei zu seiner Längsachse quergerichtete Fixierplatten 21 aus, die der Befestigung der Anordnung auf der Haut des Patienten mit Hilfe von Heftpflastern dienen und ggf. klappbar sein können.

Das in einer sterilen Verpackung befindliche, zur Anwendung bereite Punktionsbesteck 10 ist noch um eine Schutzkappe 22 ergänzt, die aus einstechbarem, d.h. weicherem, Kunststoff im Spritzgußverfahren einstückig hergestellt ist und deren eines Ende mit einem dicken Boden 23 verschlossen ist, während ihr anderes Ende eine Öffnung 24 aufweist, die klemmend auf den Fortsatz 13 des hohlen Ansatzstutzens 12 aufsteckbar ist. Die Länge des zylindrischen Hohlraumes 34 der Schutzkappe 22 zwischen der Innenfläche 25 des Bodens 23 und der Öffnung 24 ist so bemessen, daß in dem dargestellten Aufsteckzustand der Schutzkappe 22 die Spitze 17 der Stahlkanüle 16 die Innenfläche 25 des Bodens 23 nicht berührt. Der Boden 23 ist dick und einstechbar ausgebildet. Seine Tiefe entspricht minimal etwa der einfachen und maximal etwa der dreifachen Länge des schrägen Schliffes 17a der exzentrischen Spitze 17 der Stahlkanüle 16. Auf dem Außenumfang der Schutzkappe 22 sind mit gleichmäßigen Abständen längsverlaufende Versteifungsrippen 26 angeordnet. Zwischen diesen befinden sich mindestens zwei diametral gegenüberliegende Begasungsöffnungen 27, die bei dem abgebildeten Beispiel die Form länglicher Schlitze haben. Bei abgewandelten Ausführungsformen können die Begasungsöffnungen Bohrungen beliebiger Kontur sein.

Nach Abziehen der Schutzkappe 22 von dem Fortsatz 13 wird mit Hilfe der über das Kunststoffkapillar 11 vorstehenden Spitze 17 der Stahlkanüle 16 ein Blutgefäß punktiert, in das das Kunststoffkapillar 11 als Venenverweilkatheter eingesetzt werden soll. Nach erfolgreicher Punktion, die in dem Blutfängerstopfen 20 erkennbar ist, wird die Stahlkanüle 16 durch Trennung des Kanülenansatzes 15 von dem Ansatzstutzen 12 axial herausgezogen und es wird das Kunststoffkapillar 11 in das Blutgefäß soweit wie erforderlich vorgeschoben. Sodann wird die aus dem Kunststoffkapillar 11 und dem Ansatzstutzen 12 bestehende Einheit mit Hilfe der Fixierplatten 21 auf der Haut eines Patienten durch Heftpflaster befestigt.

Damit das Behandlungs-und Entsorgungspersonal sich nicht durch Stichverletzungen an der freien Stahlkanüle 16 verletzen kann, muß sie mit einer Abdeckung fest verbunden werden. Als Abdeckung für den Entsorgungsfall wird die Schutzkappe 22 verwendet, die, wie Fig. 2 zeigt, um das Stück des Ansatzstutzens 12 kürzer ist als die Stahlkanüle 16. Infolge dieser Verkürzung ist es nicht möglich, die Öffnung 24 der Schutzkappe 22 mit dem Anschlußteil des Kanülenansatzes 15 zusammenzustecken, um die Teile miteinander zu verbinden, und die Verbindung erfolgt erfindungsgemäß an dem patientenseitigen Ende der Anordnung, das in Fig. 1 eingekreist ist. Zu diesem Zweck wird die scharfe angeschliffene Spitze 17 der Stahlkanüle 16 in den dicken Boden 23 der Schutzkappe 22 eingestochen, so daß ein Festsitz erreicht wird. Die Dicke des Bodens 23 verhindert dabei, daß die Spitze 17 den Boden 23 durchdringt und zwar eine feste Verbindung zwischen Stahlkanüle 16 und Schutzkappe 22 erreicht, jedoch die Spitze nicht unschädlich gemacht wird. Die Innenfläche 25 des Bodens 23 der Schutzkappe 22 verläuft gemäß Fig. 1 und 2 im wesentlichen rechtwinklig zur Längsachse der Schutzkappe 22 und ist eben gestaltet, so daß beim Einstechen der Spitze 17 in den Boden 23 keine Führung für diese vorhanden ist und die Spitze 17 an irgendeiner Stelle in den Boden 23 eindringt. Dies ist die einfachste Ausführungsform, die sich durch verschiedene Arten der Zentrierung der Spitze 17 beim Einstechen verbessern läßt.

Solche das Einstechen der Spitze 17 erleichternden Zentrierungen ergeben sich durch die in den Fig. 2 bis 5 dargestellten Ausgestaltungen der Erfindung. Gemäß Fig. 3, 3a ist die Innenfläche 35 des dicken Bodens 33 der Schutzkappe 32 mit einer spitzen Kegelprofilierung ausgestattet, deren Spitze 36 zu der Mittelachse der Schutzkappe 32 seitlich etwas versetzt ist, um die Führung der exzentrischen Spitze 17 der Stahlkanüle 16 beim Einstechen in den Boden 33 zu erleichtern. Bei zusammengestecker Stahlkanüle 16 und Schutzkappe 32 verläuft die Stahlkanüle 16 in dem sich gegen den Boden 33 verjüngenden, im Querschnitt kreisförmigen Hohlraum 34, schräg. Dies ist jedoch für den Zusammenhalt der wegzuwerfenden Teile bedeutungslos.

Bei dem Beispiel der Fig. 4, 4a ist eine Schutzkappe 42 mit einem abgewandelten Boden 43 ausgestattet. Auch dieser Boden 43 ist dick, jedoch ist zum Erleichtern des Einstechens der Spitze 17 der Stahlkanüle 16 bei Erzielung ausreichend fester Verbindung zwischen Schutzkappe 42 und Stahlkanüle 16 eine abgewandelte Ausbildung der Innenfläche 45 des Bodens 43 vorgesehen. Die Innenfläche 45 verläuft langgestreckt kegelförmig spitz und die prismenförmige Spitze 46 des Kegels liegt auf der Mittelachse der Schutzkappe 42. Auf der langgestreckt spitz zulaufenden Innenfläche 45 des Bodens 43 sind mit gleichmäßigen gegenseitigen Abständen drei Klemmrippen 41 ausgebildet, die axial von der Spitze 46 ausgehen und deren gewölbte Rücken radial gegen die Mittelachse der Schutzkappe 42 gerichtet sind. Jede Klemmrippe 41 bildet einen radialen Vorsprung 41a und die drei Klemmrippen 41 verengen den lichten Querschnitt der von der Innenfläche 45 begrenzten Vertiefung symmetrisch zu einem Kanal. Dieser Kanal ist sowohl zur Führung der einzuschiebenden Spitze 17 der Stahlkanüle 16 als auch zu ihrer klemmenden Festhaltung nützlich. Durch weiteres Vorschieben wird die Spitze 17 der Stahlkanüle 16 in den dicken Boden 43 eingestochen und der Zusammenhalt der Teile ist optimal. Die Spitze 46 ist prismenartig profiliert, wie in Fig. 4a erkennbar ist.

Bei dem Beispiel der Fig. 5, 5a ist der Boden 53 einer Schutzkappe 52 verdickt gestaltet und mit einer exzentrisch keilförmig vertieften Profilierung der Innenfläche 55 versehen. Die Profilierung der Innenfläche 55 ergibt sich im wesentlichen durch eine konvexe Einwärtswölbung 57, die von der Innenwand einer Querhälfte der Schutzkappe 52 über die Mittelachse der Schutzkappe 52 hinaus in Richtung der Endwand 53a des Bodens 53 verläuft und die an ihrem Ende in eine zu der Mittelachse etwa senkrechte kurze Querfläche 56 übergeht, die sich an die Innenwand der anderen Querhälfte der Schutzkappe 52 anschließt. Eine in die Schutzkappe 52 eingeschobene Stahlkanüle 16 gleitet auf der Innenwand des Hohlraumes 54 der Schutzkappe 52 entlang und trifft mit der Abschrägung ihrer angeschliffenen Spitze 17 gegen die Einwärtswölbung 57 der Innenfläche 55, die sie direkt gegen die Querfläche 56 lenkt, in die die Spitze 17 einsticht. Die Stahlkanüle 16 wird so weit in den Boden 53 eingestochen, bis auch ein Stück ihres zylindrischen Teiles in dem Boden 53 steckt. Auch diese Verbindung zwischen Stahlkanüle 16 und Schutzkappe 52 ist haltbar und fest und eine für das Behandlungs-und Entsorgungspersonal gefahrlose Verwerfung der benutzten Stahlkanüle ist möglich.

**Ansprüche**

1. Punktionsbesteck, bestehend aus einem Kunststoffkapillar (11), das an seinem patientenfernen Ende einen hohlen Ansatzstutzen (12) aufweist, aus einer in das Kunststoffkapillar (11) einschiebbaren Stahlkanüle (16) mit einer angeschliffenen Spitze (17) und einem Kanülenansatz (15), der mit dem hohlen Ansatzstutzen (12) lösbar zusammensteckbar ist und aus einer hülsenartigen Schutzkappe (22) für die langgestreckten Teile, **dadurch gekennzeichnet**, daß die Schutzkappe (22) einen geschlossenen, dicken Boden (23) aufweist, in den die angeschliffene Spitze (17) der Stahlkanüle (16) klemmend einstechbar ist.

2. Punktionsbesteck nach Anspruch 1, **dadurch gekennzeichnet**, daß die Tiefe des dicken Bodens (23) minimal etwa der einfachen und maximal etwa der dreifachen Länge des Schliffes (17a) der Spitze (17) entspricht.

3. Punktionsbesteck nach Anspruch 1 oder 2, **dadurch gekennzeichnet**, daß die Innenfläche (35) des Bodens (33) kegelförmig spitz vertieft ausgebildet ist.

4. Punktionsbesteck nach Anspruch 3, **dadurch gekennzeichnet**, daß die spitze Vertiefung auf ihrer Wand mit axialen Klemmrippen (41) versehen ist, die den lichten Querschnitt der Vertiefung verengen.

5. Punktionsbesteck nach Anspruch 1 oder 2, **dadurch gekennzeichnet**, daß die Innenfläche (55) des Bodens (53) exzentrisch keilförmig vertieft ist.

6. Punktionsbesteck nach einem der Ansprüche 1 bis 5, **dadurch gekennzeichnet**, daß die Schutzkappe (22;33;43;53) im Spritzgußverfahren einteilig als rohrförmiger Körper mit äußeren axialen Versteifungsrippen hergestellt ist.

7. Punktionsbesteck nach Anspruch 6, **dadurch gekennzeichnet**, daß zwischen den Versteifungsrippen (26) in der Wand der Schutzkappe (22;33;43;53) Begasungsöffnungen (27) in Form von Schlitzen oder Bohrungen angeordnet sind.

FIG.1

FIG.2

FIG. 3

FIG.3a

FIG. 4

FIG. 4a

FIG. 5

FIG.5a

# EUROPÄISCHER RECHERCHENBERICHT

**Europäisches Patentamt**

## EINSCHLÄGIGE DOKUMENTE

EP 87107869.7

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US - A - 4 253 463 (B. KIM)<br><br>* Gesamt; insbesondere Fig. 1,2; Spalte 2, Zeilen 9-22 *<br><br>-- | 1,2 | A 61 M 5/14 |
| Y<br>A | US - A - 3 272 322 (R.W. OGLE)<br><br>* Gesamt; insbesondere Fig. 2; Spalte 1, Zeilen 44-48 *<br><br>-- | 1,2<br>3,5,6 | |
| A | DE - B - 1 228 760 (BAXTER LAB.)<br><br>* Fig. 6,10; Spalte 6, Zeile 45 - Spalte 7, Zeile 16; Spalte 9, Zeilen 51-65 *<br><br>-- | 1 | |
| A | US - A - 2 799 272 (J.H. PEACH)<br><br>* Gesamt; insbesondere Fig. 6 *<br><br>-- | 1 | |
| A | FR - A - 1 407 737 (L. NOGIER et al.)<br><br>* Gesamt; insbesondere Fig. 4 *<br><br>-- | 1 | |
| A | DE - B2 - 2 615 702 (TAUT INC.)<br><br>* Gesamt; insbesondere Fig. 3 *<br><br>-- | 1 | |
| A | DE - B2 - 2 305 896 (CH. BOEH-RINGER)<br><br>* Gesamt; insbesondere Fig. 1 *<br><br>-- | 1 | |

**RECHERCHIERTE SACHGEBIETE (Int. Cl.4)**

A 61 M 5/00

A 61 M 25/00

Der vorliegende Recherchenbericht wurde für alle Patentansprüche erstellt.

| Recherchenort | Abschlußdatum der Recherche | Prüfer |
|---|---|---|
| WIEN | 15-12-1987 | LUDWIG |

**Europäisches Patentamt**

# EUROPÄISCHER RECHERCHENBERICHT

Nummer der Anmeldung

| | EINSCHLÄGIGE DOKUMENTE | | | EP 87107869.7 |
|---|---|---|---|---|
| **Kategorie** | **Kennzeichnung des Dokuments mit Angabe, soweit erforderlich, der maßgeblichen Teile** | | **Betrifft Anspruch** | **KLASSIFIKATION DER ANMELDUNG (Int. Cl.4)** |
| A | US - A - 3 589 361 (D.A. LODER)<br><br>* Gesamt; insbesondere Fig. 1-3 *<br><br>-- | | 1 | |
| A | US - A - 3 595 230 (G.M. SUYEOKA)<br><br>* Gesamt; insbesondere Fig. 1; Spalte 2, Zeilen 64-70 *<br><br>---- | | 1 | |
| | | | | **RECHERCHIERTE SACHGEBIETE (Int. Cl.4)** |

Der vorliegende Recherchenbericht wurde für alle Patentansprüche erstellt.

| **Recherchenort** | **Abschlußdatum der Recherche** | **Prüfer** |
|---|---|---|
| WIEN | 15-12-1987 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82